(19) Europäisches Patentamt
European Patent Office
Office européen des brevets

(11) Veröffentlichungsnummer : **0 384 265 B1**

# (12) EUROPÄISCHE PATENTSCHRIFT

(45) Veröffentlichungstag der Patentschrift : **31.03.93 Patentblatt 93/13**

(51) Int. Cl.$^5$ : **C07C 317/32,** C07C 315/04

(21) Anmeldenummer : **90102765.6**

(22) Anmeldetag : **13.02.90**

## (54) Verfahren zur Herstellung von beta-Chlorethylsulfonyl-arylisocyanaten.

(30) Priorität : **16.02.89 DE 3904592**

(43) Veröffentlichungstag der Anmeldung : **29.08.90 Patentblatt 90/35**

(45) Bekanntmachung des Hinweises auf die Patenterteilung : **31.03.93 Patentblatt 93/13**

(84) Benannte Vertragsstaaten : **BE CH DE FR GB IT LI**

(56) Entgegenhaltungen :
**EP-A- 0 265 857**
**DE-A- 2 054 198**
**DE-B- 1 289 930**

(73) Patentinhaber : **HOECHST AKTIENGESELLSCHAFT**
**Postfach 80 03 20**
**W-6230 Frankfurt am Main 80 (DE)**

(72) Erfinder : **Steuernagel, Hans Helmut, Dr.**
**An den Römergärten 1**
**W-6233 Kelkheim (Taunus) (DE)**

EP 0 384 265 B1

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Erfindung betrifft ein vorteilhaftes Verfahren zur Herstellung von β-Chlorethylsulfonylarylisocyanaten der allgemeinen Formel (1)

$$Cl\text{-}H_2C\text{-}H_2C\text{-}O_2S\text{-}A\text{-}NCO \qquad (1)$$

in welcher A einen Arylenrest, wie beispielsweise einen Phenylenrest, der Substituenten aus der Reihe Alkyl($C_1$-$C_6$), Alkoxy($C_1$-$C_6$), Chlor, Brom und Nitro enthalten kann, oder einen Naphthylenrest bedeutet, indem man auf 1 Mol β-Hydroxyethylsulfonyl-arylamine der allgemeinen Formel (2)

$$HO\text{-}H_2C\text{-}H_2C\text{-}O_2S\text{-}A\text{-}NH_2 \qquad (2)$$

in welcher A die vorstehend genannte Bedeutung hat, in Gegenwart eines Katalysators mindestens 2 Mol Phosgen bei Temperaturen von etwa 100 bis etwa 200°C, vorzugsweise von etwa 130 bis etwa 180°C, gegebenenfalls in einem inerten Lösungsmittel, einwirken läßt.

Als Amine der allgemeinen Formel (2) seien beispielsweise genannt
1-Amino-4-(β-hydroxyethylsulfonyl)-benzol,
1-Amino-3-(β-hydroxyethylsulfonyl)-benzol,
1-Amino-2-methoxy-5-(β-hydroxyethylsulfonyl)-benzol,
1-Amino-2-methoxy-4-(β-hydroxyethylsulfonyl)-benzol,
1-Amino-4-methoxy-5-(β-hydroxyethylsulfonyl)-benzol,
1-Amino-2-methyl-5-(β-hydroxyethylsulfonyl)-benzol,
1-Amino-2-methyl-4-(β-hydroxyethylsulfonyl)-benzol,
1-Amino-4-methyl-5-(β-hydroxyethylsulfonyl)-benzol,
1-Amino-2-methoxy-5-methyl-4-(β-hydroxyethylsulfonyl)-benzol,
1-Amino-2,5-dimethoxy-4-(β-hydroxyethylsulfonyl)-benzol,
1-Amino-2,4-dimethoxy-5-(β-hydroxyethylsulfonyl)-benzol,
1-Amino-2-methyl-5-methoxy-4-(β-hydroxyethylsulfonyl)-benzol,
2-Chlor-1-amino-5-(β-hydroxyethylsulfonyl)-benzol,
2-Chlor-1-amino-4-(β-hydroxyethylsulfonyl)-benzol,
2-Brom-1-amino-4-(β-hydroxyethylsulfonyl)-benzol,
4-Chlor-1-amino-2-methyl-3-(β-hydroxyethylsulfonyl)-benzol,
2-Chlor-1-amino-5-methyl-4-(β-hydroxyethylsulfonyl)-benzol,
5-Chlor-1-amino-2-methoxy-4-(β-hydroxyethylsulfonyl)-benzol,
2-Nitro-1-amino-5-(β-hydroxyethylsulfonyl)-benzol,
1-Amino-5-(β-hydroxyethylsulfonyl)-naphthalin,
1-Amino-6-(β-hydroxyethylsulfonyl)-naphthalin,
2-Amino-5-(β-hydroxyethylsulfonyl)-naphthalin,
2-Amino-6-(β-hydroxyethylsulfonyl)-naphthalin und
2-Amino-8-(β-hydroxyethylsulfonyl)-naphthalin.

An geeigneten Katalysatoren seien beispielsweise genannt die Dialkyl($C_1$-$C_6$)-amide aliphatischer Carbonsäuren von 1 bis etwa 20 Kohlenstoffatomen, wie beispielsweise Dimethylformamid, Diethylformamid, Dimethyl-acetamid, Diethyl-acetamid, Diisobutyl-acetamid, Dimethyl-cocosfettsäureamid oder Diethyl-cocosfettsäureamid, ferner cyclische Verbindungen mit einer Carbonamid-Gruppierung, wie beispielsweise N-Alkyl($C_1$-$C_6$)-pyrrolidone, wie beispielsweise N-methyl-pyrrolidon oder N-Ethyl-pyrrolidon, weiterhin Phosphorsäure-tris-dialkyl($C_1$-$C_6$)-amide, wie beispielsweise Phosphorsäure-tris-dimethylamid oder Phosphorsäure-tris-diethylamid, Dialkyl($C_1$-$C_6$)-alkyl($C_{10}$-$C_{18}$)-phosphinoxide, wie beispielsweise Dimethyl-dodecyl-phosphinoxid, Tetraalkyl($C_1$-$C_6$)-harnstoffe, wie beispielsweise Tetramethylharnstoff oder Tetraethylharnstoff, und ferner tertiäre Basen, wie beispielsweise Trialkyl($C_1$-$C_6$)-amine, wie beispielsweise Trimethylamin oder Triethylamin, und Pyridin.

Der Katalysator kann vor oder nach der Zugabe des Phosgens zugesetzt werden. Er soll aber vor dem Hochheizen der Reaktionsmischung auf die Reaktionstemperatur zugegeben werden.

Es ist vorteilhaft, das Phosgen in einem Überschuß anzuwenden, wobei es zweckmäßig ist, auf 1 Mol des Amins der genannten Formel (2) etwa 2,5 bis etwa 7 Mol, besonders zweckmäßig etwa 4 bis etwa 6 Mol Phosgen einwirken zu lassen.

Das Ansetzen des Reaktionsgemisches einschließlich des Einleitens von 2 bis etwa 4 Mol Phosgen auf 1 Mol des Amins erfolgt bei Temperaturen von etwa -10° bis etwa +60°C. Anschließend wird das Reaktionsgemisch auf die beabsichtigte Reaktionstemperatur innerhalb der weiter oben angegebenen Temperaturbereiche gebracht. Im Verlauf der Hochheizphase kann zusätzliches Phosgen zugeführt werden bis zu einer maximalen Gesamtmenge von etwa 7 Mol Phosgen auf 1 Mol des Amins.

Geeignete inerte Lösungsmittel sind beispielsweise Monochlorbenzol und o-Dichlorbenzol. Führt man die

erfindungsgemäße Reaktion in einem inerten Lösungsmittel, das bei Normaldruck unterhalb der angestrebten Höchsttemperatur siedet, wie beispielsweise Monochlorbenzol bei Hochheizen auf über 132°C, durch, so arbeitet man zweckmäßigerweise in einem Druckautoklav, wobei bei geeigneter Drucksteuerung die abgespaltenen Gase (Chlorwasserstoff, Kohlendioxid) durch ein Ventil entweichen können.

Nach beendeter Reaktion wird überschüssiges Phosgen unter Durchleiten von trockener Luft oder trockenem Stickstoff durch die entstandene Isocyanatlösung oder durch Abdestillieren entfernt und zurückgewonnen.

Die entstehenden Abgase (Chlorwasserstoff und Kohlendioxid) können Phosgen mitführen, welches durch Ausfrieren aus den Abgasen zurückgewonnen werden kann.

Die entstandenen Isocyanate der genannten Formel (1), die in hoher Ausbeute anfallen, können durch restloses Abdestillieren des Lösungsmittels in Substanz isoliert werden. Je nach Löslichkeit des jeweiligen Isocyanats kann auch nach Abkühlen der gegebenenfalls auf ein geringeres Volumen eingeengten Reaktionslösung das kristallin ausgefallene Isocyanat abfiltriert werden.

Die Isocyanate der genannten Formel (1) können sowohl in Substanz als auch in Form der anfallenden Reaktionslösung, gegebenenfalls nach Einengen auf ein geringeres Volumen, für Umsetzungen eingesetzt werden.

Die Isocyanate der genannten Formel (1) sind wertvolle Ausgangsprodukte bei der Farbstoffherstellung. Ihre Verwendung für die Herstellung von Reaktivfarbstoffen ist beispielsweise in DE-AS 1 289 930 beschrieben.

Die nachfolgenden Beispiele dienen zur Erläuterung der Erfindung ohne sie darauf zu beschränken. Die darin genannten Teile sind Gewichtsteile und die Prozentangaben bedeuten Gewichtsprozente, sofern nichts anderes vermerkt ist. Gewichtsteile verhalten sich zu Volumenteilen wie das Kilogramm zum Liter.

**Beispiel 1**

In eine Suspension von 201 Teilen 1-Amino-3-(β-hydroxyethylsulfonyl)-benzol in 1950 Teilen o-Dichlorbenzol werden unter Rühren bei 0 bis 10°C 300 Teile Phosgen eingeleitet. Man läßt ohne weitere Kühlung 20 Stunden rühren, wobei die Temperatur auf 20 bis 25°C ansteigen darf. Dann fügt man 5 Teile Dimethylformamid zu und steigert die Temperatur des Reaktionsgemisches innerhalb von 4 Stunden auf 175 bis 180°C. Während des Hochheizens werden weitere 200 Teile Phosgen in das Reaktionsgemisch eingeleitet. Bei Erreichen der Siedetemperatur wird die Phosgenzufuhr abgestellt.

Man hält noch 30 Minuten bei Siedetemperatur, läßt dann abkühlen und bläst durch Einleiten von trockener Luft oder von trockenem Stickstoff die Lösung phosgenfrei. Die entstandene Lösung enthält 220 Teile des Isocyanats der Formel

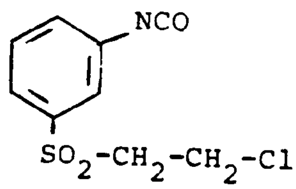

das in reinem Zustand einen Schmelzpunkt von 83°C aufweist. Es kann durch vollkommenes Abdestillieren des Lösungsmittels unter Vakuum in Substanz erhalten werden.

Man kann das entstandene Isocyanat aber auch direkt in Form der angefallenen Lösung, gegebenenfalls nach Einengen auf ein geringeres Volumen, für chemische Umsetzungen verwenden.

**Beispiel 2**

In eine Suspension von 201 Teilen 1-Amino-3-(β-hydroxyethylsulfonyl)-benzol in 1660 Teilen Chlorbenzol werden bei 0 bis 20°C 300 Teile Phosgen eingeleitet. Man läßt 6 Stunden ohne weitere Kühlung rühren, wobei die Temperatur auf 30°C ansteigen darf. Man fügt nun 5 Teile Dimethylformamid zu. Dann heizt man unter Einleiten von weiteren 300 Teilen Phosgen innerhalb von 6 Stunden auf Siedetemperatur (132°C) hoch und läßt 14 Stunden bei 132°C rühren. Das aus Chlorwasserstoff und Kohlendioxid bestehende Abgas wird durch einen tiefgekühlten (-30°C) Intensivkühler geleitet, wo mitgerissenes Phosgen kondensiert und zurückgewonnen wird. Danach wird das Reaktionsgemisch durch Destillieren bei Normaldruck von überschüssigem Phosgen befreit. Die verbleibende Lösung enthält 200 Teile Isocyanat der Formel

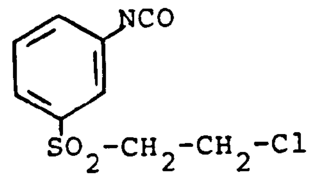

die man durch restloses Abdestillieren des Lösungsmittels in Substanz erhalten kann.

**Beispiel 3**

In einem Glasautoklav von 1000 Volumenteilen Inhalt werden 50 Teile 1-Amino-3-(β-hydroxyethylsulfonyl)-benzol in 420 Teilen Chlorbenzol verrührt. Man leitet bei 0 bis 10°C 75 Teile Phosgen ein und läßt 22 Stunden bei 10 bis 25°C rühren. Anschließend fügt man 5 Teile Dimethylformamid zu. Dann wird unter Einleiten von weiteren 70 Teilen Phosgen innerhalb von 4 Stunden die Temperatur des Reaktionsgemisches auf 125°C gesteigert, wonach die Phosgenzufuhr beendet wird. Nach Schließen der Ventile wird die Temperatur innerhalb von 1 Stunde auf 160°C erhöht und noch 4 Stunden bei 160°C gehalten.

Dabei wird das Abgasventil soweit geöffnet, daß im Autoklav ein Druck von 2,5 Bar nicht überschritten wird. Anschließend wird bei Normaldruck und 100°C solange ein trockner Stickstoffstrom durch die Lösung geleitet, bis diese frei von Phosgenresten ist. Die verbleibende Lösung enthält 200 Teile Isocyanat der Formel

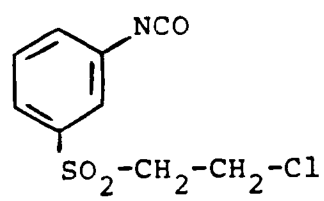

**Beispiel 4**

Ersetzt man in Beispiel 3 die 5 Teile Dimethylformamid durch 5 Teile N-Methyl-pyrrolidon und verfährt im übrigen wie in Beispiel 3 beschrieben, so erhält man eine Lösung, welche 210 Teile des Isocyanats der dort angegebenen Formel enthält.

**Beispiel 5**

Ersetzt man in Beispiel 3 die 5 Teile Dimethylformamid durch 5 Teile Pyridin und verfährt im übrigen wie in Beispiel 3 beschrieben, so erhält man eine Lösung, welche 200 Teile des Isocyanats der dort angegebenen Formel enthält.

**Beispiel 6**

Ersetzt man in Beispiel 3 die 5 Teile Dimethylformamid durch 5 Teile Dimethylacetamid und verfährt im übrigen wie in Beispiel 3 beschrieben, so erhält man eine Lösung, welche 200 Teile des Isocyanats der dort angegebenen Formel enthält.

**Beispiel 7**

Ersetzt man in Beispiel 3 die 5 Teile Dimethylformamid durch 5 Teile Diisobutyl-acetamid und verfährt in übrigen wie in Beispiel 3 beschrieben, so erhält man eine Lösung, welche 190 Teile des Isocyanats der dort angegebenen Formel enthält.

**Beispiel 8**

Ersetzt man in Beispiel 3 die 5 Teile Dimethylformamid durch 5 Teile Tetramethylharnstoff und verfährt im übrigen wie in Beispiel 3 beschrieben, so erhält man eine Lösung, welche 180 Teile des Isocyanats der dort

angegebenen Formel enthält.

**Beispiel 9**

In einem Glasautoklav von 1000 Volumenteilen Inhalt werden bei 0 bis 10°C 75 Teile Phosgen in eine vorgelegte Suspension aus 50 Teilen 1-Amino-3-(β-hydroxyethylsulfonyl)-benzol, 420 Teilen Chlorbenzol und 1,25 Teilen N-Methyl-pyrrolidon eingeleitet. Dann wird unter Einleiten von weiteren 75 Teilen Phosgen die Temperatur der Reaktionsmischung innerhalb von 4 1/2 Stunden auf 128°C gesteigert. Anschließend wird die Phosgenzufuhrt abgestellt und die Ventile werden geschlossen. Die Temperatur wird nun innerhalb einer Stunde auf 160°C erhöht und noch 4 Stunden bei 160°C gehalten. Dabei wird das Abgasventil soweit geöffnet, daß im Autoklav ein Druck von 2,5 Bar nicht überschritten wird. Darauf wird bei Normaldruck und 100°C solange trockener Stickstoff durch die Lösung geleitet, bis diese kein Phosgen mehr enthält. Die verbleibende Lösung enthält 210 Teile des Isocyanats der in Beispiel 3 angegebenenn Formel.

**Beispiel 10**

Setzt man in Beispiel 9 2,5 Teile anstatt 1,25 Teilen N-Methyl-pyrrolidon ein und verfährt im übrigen wie in Beispiel 9 beschrieben, so erhält man das gleiche Resultat wie nach Beispiel 9.

**Beispiel 11**

Ersetzt man in Beispiel 1 die 5 Teile Dimethylformamid durch 5 Teile Dimethyl-dodecyl-phosphinoxid und verfährt im übrigen wie in Beispiel 1 beschrieben, so erhält man das Isocyanat der in Beispiel 1 angegebenen Formel in ähnlich guter Ausbeute.

**Beispiel 12**

Ersetzt man in Beispiel 1 die 5 Teile Dimethylformamid durch 7 Teile Triethylamin und verfährt im übrigen wie in Beispiel 1 beschrieben, so erhält man das Isocyanat der in Beispiel 1 angegebenen Formel in ähnlich guter Ausbeute.

**Beispiel 13**

Ersetzt man in Beispiel 1 die 5 Teile Dimethylformamid durch 5 Teile Phosphorsäure-tris-dimethylamid und verfährt im übrigen wie in Beispiel 1 beschrieben, so erhält man das Isocyanat der in Beispiel 1 angegebenen Formel in ähnlich guter Ausbeute.

Ersetzt man in den Beispielen 1 bis 13 1-Amino-3-(β-hydroxyethylsulfonyl)-benzol durch die äquivalente Menge eines in Spalte 2 der folgenden Tabelle aufgeführten Amins der Formel (2) und verfährt im übrigen sinngemäß wie in Beispielen 1 bis 13 beschrieben, so erhält man die in Spalte 3 angegebenen Isocyanate der Formel (1) mit den in Spalte 4 aufgeführten Schmelzpunkten der reinen Verbindungen in ähnlichen Ausbeuten wie in Beispielen 1 bis 13 angegeben.

| Bsp. | Amin der Formel (2) ($R_1$ = $SO_2$-$CH_2CH_2$-OH) | Isocyanat der Formel (1) ($R_2$ = -$SO_2$-$CH_2CH_2$-Cl) | Schmelzpunkt |
|---|---|---|---|
| 14 | 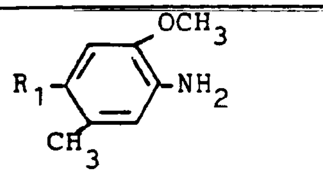 | 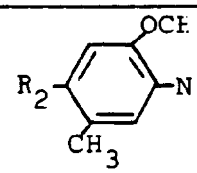 | 130°C |
| 15 | 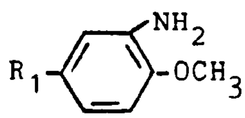 | 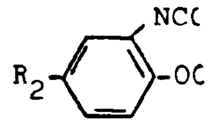 | 122°C |
| 16 | 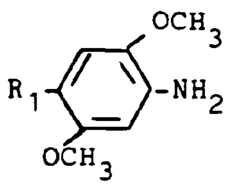 | 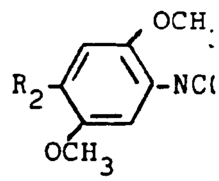 | 104°C |
| 17 | 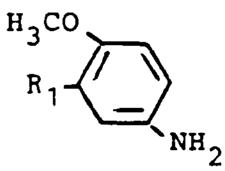 | 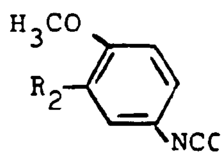 | 76°C |
| 18 | 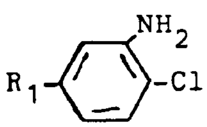 | 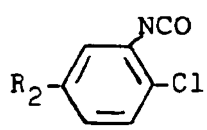 | 103°C |
| 19 | 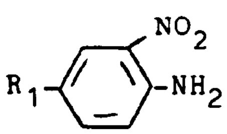 | 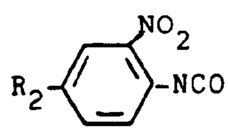 | 101°C |
| 20 | 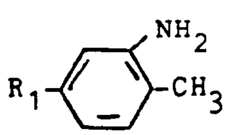 | 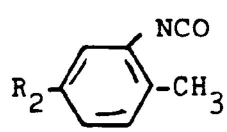 | 89°C |
| 21 | 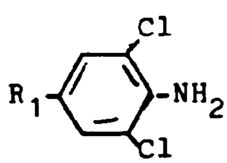 | 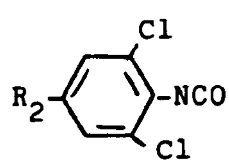 | 97°C |

| Bsp. | Amin der Formel (2) ($R_1$ = $SO_2$-$CH_2CH_2$-OH) | Isocyanat der Formel (1) ($R_2$ = -$SO_2$-$CH_2CH_2$-Cl) | Schmelzpunkt |
|---|---|---|---|
| 22 | 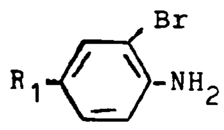 | 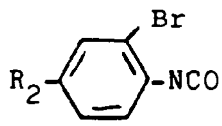 | 119°C |
| 23 | 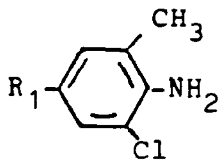 | 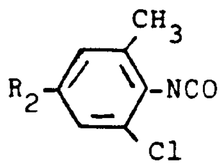 | 120°C |
| 24 | 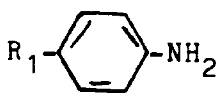 | 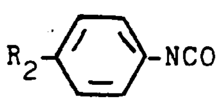 | 100°C |
| 25 | 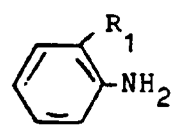 | 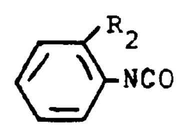 | 193°C |
| 26 | 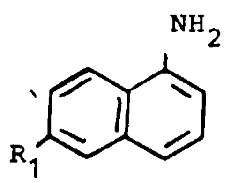 | 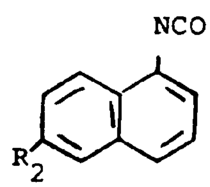 | 130°C |

## Patentansprüche

1. Verfahren zur Herstellung von β-Chlorethylsulfonylarylisocyanaten der allgemeinen Formel (1)

   $$Cl\text{-}H_2C\text{-}H_2C\text{-}O_2S\text{-}A\text{-}NCO \qquad (1)$$

   in welcher A einen Arylenrest, der Substituenten aus der Reihe Alkyl($C_1$-$C_6$), Alkoxy($C_1$-$C_6$), Chlor, Brom und Nitro enthalten kann, oder einen Naphthylenrest bedeutet, dadurch gekennzeichnet, daß man auf 1 Mol eines β-Hydroxyethylsulfonyl-arylamins der allgemeinen Formel (2)

   $$HO\text{-}H_2C\text{-}H_2C\text{-}O_2S\text{-}A\text{-}NH_2 \qquad (2)$$

   in welcher A die vorstehend genannte Bedeutung hat, in Gegenwart eines Katalysators mindestens 2 Mol Phosgen bei Temperaturen von etwa 100 bis etwa 200°C, gegebenenfalls in einem inerten Lösungsmittel, einwirken läßt.

2. Verfahren nach Anspruch 1, dadurch gekennzeichnet, daß man ein Dialkyl($C_1$-$C_6$)-amid aliphatischer Carbonsäuren von 1 bis etwa 20 Kohlenstoffatomen, ein N-Alkyl($C_1$-$C_6$)-pyrrolidon, ein Phosphorsäure-tris-dialkyl($C_1$-$C_6$)-amid, ein Dialkyl($C_1$-$C_6$)-alkyl($C_{10}$-$C_{18}$)-phosphinoxid, einen Tetraalkyl($C_1$-$C_6$)-harnstoff, ein Trialkyl($C_1$-$C_6$)-amin oder eine cyclische tertiäre Base als Katalysator verwendet.

3. Verfahren nach mindestens einem der Ansprüche 1 und 2, dadurch gekennzeichnet, daß man Dimethylformamid, Diethylformamid, Dimethyl-acetamid, Diethyl-acetamid, Diisobutyl-acetamid, Dimethyl-cocosfettsäureamid, Diethyl-cocosfettsäureamid, N-Methylpyrrolidon, N-Ethylpyrrolidon, Phosphorsäure-tris-dimethylamid, Phosphorsäure-tris-diethylamid, Dimethyl-dodecyl-phosphinoxid, Tetramethylharnstoff, Tetraethylharnstoff, Trimethylamin, Triethylamin oder Pyridin als Katalysator verwendet.

4. Verfahren nach mindestens einem der Ansprüche 1 bis 3, dadurch gekennzeichnet, daß man das Phosgen auf das Amin bei Temperaturen von etwa 130 bis etwa 180°C einwirken läßt.

5. Verfahren nach mindestens einem der Ansprüche 1 bis 4, dadurch gekennzeichnet, daß man in Monochlorbenzol oder o-Dichlorbenzol als inertem Lösungsmittel umsetzt.

6. Verfahren nach mindestens einem der Ansprüche 1 bis 5, dadurch gekennzeichnet, daß man bei Umsetzung in einem inerten Lösungsmittel, das unterhalb 180°C bei Normaldruck siedet, in einem Druckautoklav arbeitet.

## Claims

1. A process for the preparation of a β-chloroethylsulfonylaryl isocyanate of the formula (1)

$$Cl\text{-}H_2C\text{-}H_2C\text{-}O_2S\text{-}A\text{-}NCO \quad (1)$$

in which A is an arylene radical, which may contain substituents from the series comprising alkyl ($C_1$-$C_6$), alkoxy ($C_1$-$C_6$), chlorine, bromine and nitro, or a naphthylene radical, which comprises allowing at least 2 moles of phosgene to act on 1 mole of a β-hydroxyethylsul fonyl-arylamine of the formula (2)

$$HO\text{-}H_2C\text{-}H_2C\text{-}O_2S\text{ -}A\text{-}NH_2 \quad (2)$$

in which A has the abovementioned meaning, in the presence of a catalyst at temperatures of about 100 to about 200°C, if appropriate in an inert solvent.

2. The process as claimed in claim 1, wherein a dialkyl($C_1$-$C_6$)-amide of an aliphatic carboxylic acid having 1 to about 20 carbon atoms, an N-alkyl($C_1$-$C_6$)-pyrrolidone, a phosphoric acid tris-dialkyl($C_1$-$C_6$)-amide, a dialkyl($C_1$-$C_6$)-alkyl ($C_{10}$-$C_{18}$)-phosphine oxide, a tetraalkyl ($C_1$-$C_6$)-urea, a trialkyl ($C_1$-$C_6$)-amine or a cyclic tertiary base is used as the catalyst.

3. The process as claimed in at least one of claims 1 and 2, wherein dimethylformamide, diethylformamide, dimethyl-acetamide, diethyl-acetamide, diisobutylacetamide, coconut fatty acid dimethylamide, coconut fatty acid diethylamide, N-methylpyrrolidone, N-ethylpyrrolidone, phosphoric acid tris-dimethylamide, phosphoric acid tris-diethylamide, dimethyl-dodecylphosphine oxide, tetramethylurea, tetraethylurea, trimethylamine, triethylamine or pyridine is used as the catalyst.

4. The process as claimed in at least one of claims 1 to 3, wherein the phosgene is allowed to act on the amine at temperatures of about 130 to about 180°C.

5. The process as claimed in at least one of claims 1 to 1, wherein the reaction is carried out in monochlorobenzene or o-dichlorobenzene as the inert solvent.

6. The process as claimed in at least one of claims 1 to 5, wherein if the reaction is carried out in an inert solvent having a boiling point, under normal pressure, below 180°C, a pressure autoclave is used.

## Revendications

1. Procédé de préparation d'isocyanates de β-chloroéthylsulfonylaryle de formule générale (1)

$$Cl\text{-}H_2C\text{-}H_2C\text{-}O_2S\text{-}A\text{-}NCO \quad (1)$$

dans laquelle A représente un reste arylène qui peut contenir des substituants du groupe constitué par alkyle en $C_1$-$C_6$, alcoxy en $C_1$-$C_6$, chlore, brome et nitro, ou un reste naphtylène, caractérisé en ce qu'on fait réagir en présence d'un catalyseur, à des températures d'environ 100 à environ 200°C, au moins 2 moles de phosgène sur 1 mole d'une β-hydroxyéthylsulfonylarylamine de formule générale (2)

$$HO\text{-}H_2C\text{-}H_2C\text{-}O_2S\text{-}A\text{-}NH_2 \quad (2)$$

dans laquelle A a la signification indiquée précédemment, éventuellement dans un solvant inerte.

2. Procédé selon la revendication 1, caractérisé en ce qu'on utilise comme catalyseur un di(alkyl en $C_1$-$C_6$)amide d'acides carboxyliques aliphatiques de 1 à environ 20 atomes de carbone, une N-(alkyl en $C_1$-

$C_6$)pyrrolidone, un tris-di (alkyl en $C_1$-$C_6$)amide d'acide phosphorique, un oxyde de di(alkyl en $C_1$-$C_6$)-(alkyl en $C_{10}$-$C_{18}$)phosphine, une tétra(alkyl en $C_1$-$C_6$)urée, une tri(alkyl en $C_1$-$C_6$)amine ou une base tertiaire cyclique.

3. Procédé selon au moins l'une des revendications 1 et 2, caractérisé en ce qu'on utilise comme catalyseur le diméthylformamide, le diéthylformamide, le diméthylacétamide, le diéthylacétamide, le diisobutylacétamide, le diméthylamide d'acide gras de coco, le diéthylamide d'acide gras de coco, la N-méthylpyrrolidone, la N-éthylpyrrolidone, le tris-diméthylamide de l'acide phosphorique, le tris-diéthylamide de l'acide phosphorique, l'oxyde de diméthyldodécylphosphine, la tétraméthylurée, la tétraéthylurée, la triméthylamine, la triéthylamine ou la pyridine.

4. Procédé selon au moins l'une des revendications 1 à 3, caractérisé en ce que l'on fait réagir le phosgène sur l'amine à des températures d'environ 130 à environ 180°C.

5. Procédé selon au moins l'une des revendications 1 à 4, caractérisé en ce que l'on effectue la réaction dans du monochlorobenzène ou de l'o-dichlorobenzène comme solvant inerte.

6. Procédé selon au moins l'une des revendications 1 à 5, caractérisé en ce que, pour une réaction dans un solvant inerte qui bout au-dessous de 180°C sous la pression normale, on travaille dans un autoclave à pression.